# EUROPEAN PATENT APPLICATION

(11) **EP 1 314 359 A1**
(43) Date of publication of application: **28.05.2003**
(21) Application number: 01870253.0
(22) Date of filing: 21.11.2001
(51) Int. Cl.: A23K 1/16, A61K 35/84

(54) **Growth promoter composition for animals**

(71) Applicant: N.V. Seghers Nutrition Sciences, 9031 Drongen (BE)
(72) Inventor: MOLLY, Koen, 9870 Olsene (BE); BRUGGEMAN, Geert, 8200 Brugge (BE)
(74) Representative: Brants, Johan Philippe Emile

(57) **Abstract**

The present invention relates to a composition comprising a fungus and at least one growth-promoting component selected from the group comprising organic acids, inorganic acids, animal feed antibiotics, conventional growth promoters, and plant extracts.

The invention further relates to the use of said composition as an antimicrobial agent and to an animal feed comprising said composition and to a method for the improvement of growth and/or for reducing feed conversion and/or for improving feed value and/or for improving health and well-being of an animal by providing said animal with a feed comprising a composition.

## Description

### Technical field

The present invention relates to a growth promoter composition suitable for animals. The growth promoter composition according to this invention is useful as an animal feed. The primary object is the improvement of the microbial ecosystem in the gastrointestinal tract resulting in an optimization of the feed conversion ratio.

The present invention discloses further a process for improving the efficiency of feed utilization and/or for promoting the growth of animals in which the animal is fed a diet which comprises said growth promoter composition.

### Background of the invention

The well being and health and hence the economic value of animals such as livestock depends on a good-functioning and well-balanced microbial ecosystem in the gastrointestinal tract of the host. Disturbance of this microbial eco-system by e.g. bacterial infection will ultimately result in a decreased performance of the animal, often attended with infectious diarrhea. As a consequence, the feed taken up by the host is not efficiently converted.

A low FCR (Feed Conversion Ratio is the ratio of the amount of feed consumed relative to the weight gain of an animal) indicates that a given amount of feed results in a growing animal gaining proportionately more weight. The latter situation indicates that the animal is able to utilize the feed more efficiently.

One way in which the FCR of a feed is lowered is to influence the bio-regulatory process through administering traditional antimicrobials or feed antibiotics. Those skilled in the art appreciate, however, that antibiotic therapy for the treatment of numerous disorders and diseases results in the destruction of the intestinal microflora. It is necessary to develop and use new antibiotics and mixtures thereof to the animals as described in e.g. EP Patent 597167 which discloses the use of an antibiotic mixture of gentamycin and lincomycin.

An important draw-back, however is that many antibiotics applied to the animals provoke resistance of the bacteria present in the animals and other microorganisms. Further, a number of the used antibiotics to the animals are the same or chemically very closely related to those antibiotics which are used in the fight against diseases provoked by bacteria and other micro-organisms in humans. As such, there has been an increasing concern in the public opinion that the resistance found in the microorganisms in the livestock host should be transferred to the disease provoking microorganisms living in human beings, wherefore these will not be fightable with the present human antibiotics at disposal.

Alternative feed additives to improve the animal's well-being and hence its ability to convert feed into food products are increasingly being used and include feed acidifiers for improved protection of the digestive tract against proliferation of pathogenic bacteria (WO Patent 98/43634). Also very different types of additives such as fungi [e.g. *Lentinus edodes* (Shiitake)] have been reported for use in feedstock (US Patent No. 4,711,787) although no improved feed conversion has been demonstrated.

With regard to the relative expensive cost factor of feed in the production of food-producing animals, it is clear that an additional aspect of lowered feed conversions will directly improve the profitability of a feed producer.

It is an object of the present invention to provide a composition which provides a low feed conversion ratio (FCR) without increasing the feed cost per unit weight. It is a further object to the present invention to provide a composition which enhances growth. It is a further object of the present invention to provide a composition which improves the microbial balance of the intestinal microflora. It is a further object of the present invention to provide a method of enhancing weight gain and feed efficiency in an animal.

### Summary of the invention

The invention relates in general to a composition comprising a fungus and at least one additional growth promoting compound selected from the group comprising organic acids, inorganic acids, animal feed antibiotics, conventional growth promoters, and plant extracts.

The invention further relates to methods for improving growth and/or reducing feed conversion and/or for improving feed value and/or for improving health and well-being of an animal by providing said animal with a feed comprising a composition according to the present invention.

### Detailed description of the invention

Unless defined otherwise, all technical and scientific terms used herein have the same meaning as commonly understood to one of ordinary skill in the art.

In this specification and the appended claims, the singular forms "a", "an", and "the" include plural references unless dictated otherwise.

The present invention relates to improved feed conversion activities of animals without concomitant substantial or significant stimulation of feed intake. Typically the feed intake is the same or decreased in treated animals as compared to untreated animals. New growth-promoting compositions and methods for obtaining the above are provided.

As used herein the expression "improving the feed conversion" and cognate expressions such as "feed conversion improvement" refer to improving feed utilization efficiency and/or improving growth rate. That is, in accordance with the present invention, treated animals (as compared to untreated animals) can have substantially the same feed intake and grow at an increased growth rate, can have decreased feed intake and grow at substantially the same growth rate, or can have decreased feed intake and grow at an increased growth rate. The term "growth" as used herein refers to a gain in weight.

In accordance with the present invention, in a first aspect, a composition is provided which comprises a fungus and at least one growth-promoting component selected from the group comprising organic acids, inorganic acids, animal feed antibiotics, conventional growth promoters, and plant extracts. Said growth-promoting component may be a single organic acid, a single inorganic acid, a single animal feed antibiotic, a single conventional growth promoter, a mixture of organic acids, a mixture of inorganic acids, a mixture of animal feed antibiotics, a mixture of conventional growth promoters, a mixture of organic acids and inorganic acids, a mixture of organic acids and animal feed antibiotics, a mixture of organic acids and conventional growth promoter, a mixture of inorganic acids and animal feed antibiotics, a mixture inorganic acids and conventional growth promoters, a mixture of animal feed antibiotics and conventional growth promoters, a mixture of inorganic acids, organic acids, animal feed antibiotics and conventional growth promoters. As used herein, the term "growth-promoting component" refers to a compound or composition which exerts a growth-promoting activity or effect within a living vertebrate host, which excludes fungi. Said growth-promoting component is usually (part of) a feed additive or feed supplement.

Additional useful growth-promoting components include but are not limiting to prebiotics such as oligosaccharides including inulins, fructo-oligosaccharides, and galacto-oligosaccharides; probiotics such as *Bifidobacterium thermophilum* and *infantis, Lactobacillus acidophilus, bulgaricus, casei, lactis, plantarum,* and *reuteri, Bacillus subtilis* and *cereus, Streptococcus faecium, diacetilactus,* and *thermophilus, Enterococcus faecium* and *faeclum, Torulopsia, Aspergillus oryzae,* and *Streptomyces;* including their vegetative spores and synthetic derivatives; synbiotics; enzymes such as acid proteases from *Rhizopus rhizopodiformis, Aspergillus niger,* and the genus *Tramates,* and neutral to alkaline proteases from *Bacillus subtilis, licheniformis,* and *natta;* and herbs such as *Mimosaceae* and *oregano,* including extracts and essential oils thereof may be used as a growth-promoting components. Suitable species are further: Lawsonia sp. en Serpulina sp.

The combination of fungus with a growth-promoting component as provided by the new compositions according to the present invention has a synergistic effect on feed conversion improvement leading to much reduced FCRs.

Compared to FCR's obtained with single-growth-component feed additives, compositions of the present invention provide substantially higher feed conversion and substantially lower FCR. As used herein, the term "single-growth-component feed additive" refers to an additive comprising at least one growth component within a single class of growth-promoting components. The term "class" of growth-promoting components as used herein refers to a group or set of growth components sharing common characteristics and/or functionalities such as e.g. class of antibiotics, class of prebiotics, class of probiotics, class of synbiotics, class of herbs, class of conventional growth promoters, class of organic acids, and class of inorganic acids.

In one embodiment of the present invention, the growth promoter component may be a single compound or a mixture of compounds within a single class of growth-promoting components or mixtures of single or two or more compounds from a single or two or more classes.

In one embodiment of the present invention, a composition is provided wherein the growth-promoting component is at least one component selected from the group comprising organic acids and inorganic acids. Accordingly, the growth-promoting component of the feed additive according to the present invention may be a single organic acid, a single inorganic acid, a mixture of organic acids, a mixture of inorganic acids or a mixture of organic and inorganic acids.

In a further embodiment, a composition is provided wherein the growth-promoting component is an organic acid. In a further embodiment, a composition is provided wherein the growth-promoting component is an organic acid being a C₁₋₁₂ carboxylic acid selected from the group comprising unsubstituted carboxylic acids such as formic acid, acetic acid, propionic acid, butyric acid, valerianic acid, and caproic acid, substituted carboxylic acids such as adipic acid, maleic acid, succinic acid, citric acid, fumaric acid, tartaric acid, lactic acid, gluconic acid, malic acid, and ascorbic acid, including cyclic carboxylic acids such as picolinic acid. The organic acid component may be a single unsubstituted carboxylic acid, a single substituted carboxylic acid, a mixture of unsubstituted carboxylic acids, a mixture of substituted carboxylic acids and a mixture of unsubstituted carboxylic acids and substituted carboxylic acids including saturated, unsaturated, cyclic and aliphatic carboxylic acids and metal complexes and salts thereof. Also single racemic forms and racemic mixtures may be used.

In a further embodiment, when added in feed for animals, useful concentrations of such carboxylic acids range from 0.05 eq/kg to 0.25 eq/kg feed. In a further embodiment, concentrations of such carboxylic acids range from 0,10 eq/kg to 0.20 eq/kg feed. In a further embodiment, concentrations of such carboxylic acids range from 0,10 eq/kg to 0.15 eq/kg feed. In yet a further embodiment, concentrations of such carboxylic acids range from 0.12 eq/kg to 0.18 eq/kg feed.

In yet a further embodiment, a composition is provided wherein the growth-promoting component is an inorganic acid including strong inorganic acids in small quantities such as perchloric acid, hydroiodic acid, hydrobromic acid, hydrochloric acid, sulfuric acid, and nitric acid and weak inorganic acids such as phosphoric acid, hydrofluoric acid, hypochlorous acid, and nitrous acid.
Suitable concentrations of weak and strong inorganic acids which are useful range from 0.1-0.3% to 1-1.5% by weight.

In another embodiment according to the present invention, a composition is provided wherein the growth-promoting component is an animal feed antibiotic. Non-limiting examples of useful animal feed antibiotics include avoparcin, bacitracin such as zinc bacitracin, chlortetracyclin, flavophospholipol (flavomycin), furadox, gentamicin, lasalocid, lincomycin, monensin, neomycin, oleandomycin, oxytetracyclin, sarimomoicin, spiramycin, sulfametazine, tetracycline, tylosin, virginiamycin. In a further embodiment, useful concentrations of an animal feed antibiotic ranges from 0.5 ppm animal feed to 250 ppm animal feed. In a further embodiment, useful antibiotic concentrations for animal feed include from 1 ppm to 150 ppm. In a further embodiment, useful antibiotic concentrations for animal feed include from 2 ppm to 100 ppm. In a further embodiment, useful antibiotic concentrations for animal feed include from 2.5 ppm to 50 ppm. In yet a further embodiment, useful antibiotic concentrations for animal feed include from 2.5 ppm to 5 ppm.

In another embodiment according to the present invention, a composition is provided wherein the growth-promoting component is a conventional growth promoter selected from the group comprising nitrovin, olaquindox, carbadox, and cyadox. Concentrations of such conventional growth promoters ranges from 20 ppm to 150 ppm but usually range from 50 ppm to 100 ppm.

In another embodiment of the present invention a composition is provided wherein the fungus is a basidiomycete. As used herein, the term "basidiomycetes" refers to mushrooms and macrofungi belonging to the class Basidiomycotina, producing basidiospores. Non-limiting examples of useful basidiomycetes include genera belonging to the orders Agaricales and Aphyllophorales, such as *Armillariella mellea* (Fr.) Karst, *Tricholoma matsutake* (*S.* Ito et Imai) Sing., *Lentinus edodes* (Berk.) Sing. and Shiitake, *Coriolus versicolor* (Fr.) Quel., *Grifola gigantea* (Fr.) Pilat, Favolus Arcularius (Fr.) Ames, Ganoderma (Reishi), Cordyceps, Coriolus or Grifola (Maitake), either alone or in any combination with each other.
In a particular useful embodiment of the present invention, a composition is provided wherein the fungus is *Lentinus edodes,* (Berk.) Shiitake.

In a further embodiment, a composition is provided wherein the fungus is in the form of an extract, a derivative, a dried status or a crude status including any mixture of said forms.
A particular useful embodiment provides a composition wherein the fungus is a crude component selected from the group comprising spores, mycelium, fruiting bodies, products isolated from cultivated fermentation broths, and entire mashed organisms, either as a single component or in any combination with each other.

Accordingly, the use of a composition according to present invention has been proven to be particularly suitable for lowering feed conversion ratio's and/or growth enhancement.
In a further embodiment, the compositions of the present invention may be suitable for intestinal function improvement and/or carcass quality improvement. In a further embodiment, when fed to dairy animals such as dairy cows, goats and ewes, the feed additive composition of the present invention may improve milk production.

In a further embodiment, a composition according to the present invention comprises an amount from 50% to 99.9% by weight of fungus. Said fungus being basidiomycetes of which particular useful but non-limiting examples include genera belonging to the orders Agaricales and Aphyllophorales, such as Armillariella mellea (Fr.) Karst, Tricholoma matsutake (S. Ito et Imai) Sing., Lentinus edodes (Berk.) Sing. and Shiitake, Coriolus versicolor (Fr.) Quel., Grifola gigantea (Fr.) Pilat, Favolus Arcularius (Fr.) Ames, Ganoderma (Reishi), Cordyceps, Coriolus or Grifola (Maitake), either alone or in any combination with each other; particularly useful are fungi being Lentinus edodes (Berk.) Shiitake. In a further embodiment, an amount from 70% to 99.9% by weight of fungus is comprised in a composition according to the present invention. In a further embodiment, an amount from 85% to 99.9% by weight of fungus is comprised in a composition according to the present invention. In yet a further embodiment, an amount from 95% to 99.9% by weight of fungus is comprised in a composition according to the present invention.

According to this specification, a composition is provided comprising a fungus and at least one growth-promoting component. It is generally recognized by a skilled person in the art that supplemental ingredients are being used, e.g. various additives such as amino acids, minerals, vitamins, enzymes, etc., with various objects, such as nutrient replenishment, nutrient fortification, improvements of digestion and absorption, disease prevention, etc.

In a further embodiment, a composition according to the present invention is particularly useful as a feed additive, particularly in livestock feed. In a further embodiment, a composition according to the present invention is particularly useful for animal feed. In a further embodiment, a composition according to the present invention is particularly useful for poultry and pig feed.

Accordingly, the use of a composition according to present invention has been proven to be particularly suitable for lowering feed conversion ratio's and/or growth enhancement.
Further, a composition of the present invention may be suitable for intestinal function improvement and/or carcass quality improvement. When fed to dairy animals such as dairy cows, goats and ewes, a composition of the present invention may improve milk production.

It is a second aspect of the present invention to provide an animal feed comprising a composition according to the present invention from 0.01% to 20% by weight and from 80% to 99.999% by weight standard animal feed. As used herein, the term "standard animal feed" refers to feed for meat-producing animals, i.e. feed that can be used in the animal husbandry field and is suitable to be fed to meat-producing animals to supply part or all of the meat-producing animal's nutrient requirements. Main ingredients of standard animal feed include but are not limited to wheat flour, starch, dextrin, cereal grains such as corn, milo, etc., oil seed meals, such as soybean meal, rapeseed meal, cottonseed meal, linseed meal, chaffs and brans, such as rice bran, de-oiled rice bran, wheat bran, fish meals, oils and fats, such as beef tallow, soybean oil, palm oil, coconut oil, and fish oil.

In a further embodiment, said animal feed comprises a composition according to the present invention from 0.1% to 10% by weight. In a further embodiment, said animal feed comprises a composition according to the present invention from 0.5% to 5% by weight. In a further embodiment, said animal feed comprises a composition according to the present invention from 0.1% to 5% by weight. In a further embodiment, said animal feed comprises a composition according to the present invention from 0.5% to 2% by weight.

According to the present invention, an animal feed is provided for use as feed for meat-producing animals. As herein used, the term "meat-producing animals refer to livestock animals including but not limited to cattle such as ruminants, sheep, swine including pigs and hogs, horses, and poultry, and their progeny, such as sucking pigs, piglets, calves, lambs kids, foals and chickens etc.
Also fishes, such as eel, carps, trout, rainbow trout, yellowtail, sea bream, silver salmon, gold fishes, colored carp, tropical fishes; shellfishes; crustaceans; are examples of the animals covered by the composition and/or animal feed of the present invention.
Also pets, such as dogs, cats, rabbits, hamsters, and their progeny are examples of the animals covered by the composition and/or animal feed of the present invention.

In a further embodiment, an animal feed according to the present invention is used for poultry or pig feed, in particular during the early life stages of said poultry and pigs. Non-limiting examples of poultry that may be considered in the present invention include chickens, ducks, geese, guinea fowl, peafowl, pigeons, and turkeys. As used herein, the wording "during early life stage of poultry" refers to a life stage comprised between 1 day and 4 weeks. As used herein, the wording "during early life stage of pig" refers to a life stage comprised between 1 week before and 1 week after weaning. In a preferred embodiment an animal feed according to the invention is fed to piglets during a period comprised between weaning and up to 20 kg. In a further preferred embodiment an animal feed according to the invention is fed to poultry during a period comprised between 1 day and 4 weeks.

A further aspect of the present invention relates to a method for the improvement of growth and/or for reducing feed conversion and/or for improving feed value and/or for improving health and well-being of an animal by providing said animal with a feed comprising a composition as provided in this specification.

The composition of the present invention may be added either directly to the animal's feed or may be added as a premix. The composition according to the present invention will mostly be present in a form of a dry powder or mini-granules but may also be present in humidified, pasta-like emulsion-like or liquid form. A powder additive may be prepared by dryblending of the ingredients, but it may also be prepared by wet-blending of the ingredients followed by drying, grinding and possible sifting. A mini-granulate may be prepared the same way although to begin with, a sufficient amount of liquid may be added of an appropriate kind (e.g. water) in order to obtain sufficient adhesion power between the particles of the ingredients.

Accordingly, the present invention relates to induction by feeding the animal with the compositions of the present invention of changes in the microbial ecosystem in the gastrointestinal tract of the animal in a specific way resulting in an improved gastrointestinal ecosystem. The total amount of enteric pathogens are in a first stage enumerated in the gastrointestinal tract, i.e. a selective development of the enteric pathogens within the gastrointestinal tract, and in a second stage, the enumerated enteric pathogens are very quickly excreted from the gastrointestinal tract of the animal. As used herein, the term "enteric pathogens" refers to an undesired population of pathogens. As will be appreciated, there are a large number of such pathogens including but not limiting to Gram negative bacteria like Salmonella, Escherichia, Shigella, Klebsiella, Erwinia, Yersinia, Campylobacter, Helicobacter, Vibrio, and Pseudomonas; Gram positive bacteria like Clostridium; viruses like Norwalk virus, Norwalk-like viruses and Rotavirus; and protozoa like Crytosporidium, Entamoeba, Giardia, and Dientamoeba. The present invention would be suitable for the suppression of these pathogens in the gastrointestinal tract of animals.

By fast elimination of enteric pathogens from the gastrointestinal tract, in a second instance, better performances, which reflect in daily growth and feed conversion of the treated animals are obtained. The overall growth promoting affect of the composition of the present invention is readily and clearly visible at the elution level of the enriched enteric pathogen population from the gastrointestinal tract of the animal.

The administration of the composition according to the present invention to animals thus allows for a specific sequential action, i.e. the enumeration of the enteric pathogens in the gastrointestinal tract of the animal prior to a wash-out of the enumerated enteric pathogens. As a result of the synergistic action of the composition of the present invention, far less diarrhea and much improved performances are obtained.

### Examples

The following examples of the invention are exemplary and should not be taken as in any way limiting.

### Example 1: Influence of Lentinus edodes on the zootechnic performance of poultry

20 x 40 one day old chickens were provided with following feeds: control feed, control feeds supplemented with a growth-promoting component, control feed supplemented with 0,5 % fungi (*Lentinus edodes*) and control feed supplemented with a growth-promoting component and with 0,5 % fungi (*Lentinus edodes*). As used herein, the term "control feed" refers to a standard animal feed for meat-producing animals, i.e. feed that can be used in the animal husbandry field and is suitable to be fed to meat-producing animals to supply part or all of the meat-producing animal's nutrient requirements. Water and feed were supplied *ad libitum.* At the end of the trial, chickens were weighed and feed conversion ratio's were determined (Table 1). Microbial contents in the gastrointestinal tract were determined by plate counting.

**Table 1.**

| Influence of *Lentinus edodes* on chicken performance. The indications "+" and "-" indicate qualitative values. | | |
|---|---|---|
| Diet | Daily growth | FCR |
| control | = | = |
| control + MCFA | ++ | -- |
| control + formic acid | + | - |
| control + phophorous acid | + | - |
| control + Salinomycine | + | - |
| control + Amoxyciline | + | - |
| control + inulin | + | - |
| control + Bacillus sp. | + | - |
| control + Oregostim | + | - |
| control + *Lentinus edodes* | *++* | *--* |
| control + Aromabiotic + *Lentinus edodes* | *++++* | *----* |
| control + formic acid + *Lentinus edodes* | *++++* | *----* |
| control + phophorous + *Lentinus edodes* | ++++ | ---- |
| control + Salinomycine + *Lentinus edodes* | ++++ | ---- |
| control + Amoxyciline + *Lentinus edodes* | *++++* | *----* |
| control + inulin + *Lentinus edodes* | *++++* | *----* |
| control + Bacillus sp. + *Lentinus edodes* | *++++* | *----* |
| control + Oregostim + *Lentinus edodes* | *++++* | *----* |

From Table 1, it is clear that the combination of the use of *Lentinus edodes* with a growth-promoting component has a synergistic effect on growth and feed conversion ratio of the animal. We conclude that compared to FCR's obtained with single-growth-component feed additives, compositions of the present invention provides substantially higher feed conversion and substantially lower FCR.

**Table 2.**

| Synergistic effect of *Lentinus edodes* on MCFA for chicken performance. *, *Lentinus edodes* | | | | |
|---|---|---|---|---|
| | Diet | | | |
| | Control | Control + Aromabiotic | Control + LE* | Control + MCFA + LE* |
| Growth (mg) / chicken at week 7 | 1738 | 1766 | 1733 | **1830** |
| Feed conversion / chicken | 1,586 | 1,628 | 1,630 | 1,639 |

Table 2 shows that the use of fungi (*Lentinus edodes*) in combination with MCFA results in a significant increase in chicken growth per chicken while the FCR decreased.

## Claims

1. A composition comprising a fungus and at least one growth-promoting component selected from the group comprising organic acids, inorganic acids, animal feed antibiotics, conventional growth promoters, and plant extracts.

2. A composition according to claim 1, wherein the growth-promoting component is selected from the group comprising organic acids and inorganic acids.

3. A composition according to claim 2, wherein the growth-promoting component is an organic acid.

4. A composition according to claim 3, wherein the organic acid is a carboxylic acid selected from the group comprising C₁₋₈ substituted, unsubstituted, saturated, and unsaturated carboxylic acids.

5. A composition according to any previous claims 1-4, wherein the growth-promoting component is an animal feed antibiotic.

6. A composition according to any previous claims 1-5, wherein the growth-promoting component is a conventional growth promoter selected from the group comprising nitrovin, olaquindox, carbadox, and cyadox.

7. A composition according to any of previous claims 1-6, wherein the fungus is selected from genera within the Basidomycetes.

8. A composition according to claim 7, wherein the fungus is selected from genera belonging to the order Agaricales or Aphyllopharales of Homobasidae, such as for example *Armillariella mellea* (Fr.) Karst, *Tricholoma matsutake* (S. Ito et Imai) Sing., *Lentinus edodes* (Berk.) Sing. or Shiitake, *Coriolus versicolor* (Fr.) Quel., *Grifola gigantea* (Fr.) Pilat, *Favolus Arcularius* (Fr.) Ames, Ganoderma (Reishi), Cordyceps, Coriolus or Grifola (Maitake), either alone or in combination with each other.

9. A composition according to claim 8, wherein the fungus is *Lentinus edodes,* (Berk.) Shiitake.

10. A composition according to claim 9, wherein the fungus is in a crude form comprising spores, mycelium, fruiting bodies, products isolated from cultivated fermentation broths, and entire mashed organisms.

11. A composition according to any of previous claims 1-10 comprising fungus in an amount from about 50% to about 99.9% by weight.

12. Use of a composition according to any of the previous claims 1-11 as an animal feed additive.

13. Use of a composition according to any of the previous claims 1-11 for lowering the feed conversion ratio.

14. Use of a composition according to any of previous claims 1-11 as poultry or pig feed, in particular during the early life stages.

15. An animal feed comprising a composition according to any previous claims 1-11 in an amount from about 0.01% to about 20% by weight.

16. A method for the improvement of growth and/or for reducing feed conversion and/or for improving feed value and/or for improving health and well-being of an animal by providing said animal with a feed comprising a composition according to any of the previous claims 1-9.
